# EUROPÄISCHE PATENTSCHRIFT

(11) **EP 1 395 300 B1**
(45) Veröffentlichungstag und Bekanntmachung des Hinweises auf die Patenterteilung: **16.03.2005**
(21) Anmeldenummer: 02747224.0
(22) Anmeldetag: 14.06.2002
(51) Int. Cl.: A61L 27/32, A61L 27/42, A61L 27/30

(54) **OBERFLÄCHENBEHANDELTES METALLISCHES IMPLANTAT UND STRAHLGUT**
SURFACE TREATED METALLIC IMPLANT AND BLASTING MATERIAL
IMPLANT METALLIQUE TRAITE EN SURFACE ET PRODUIT DE GRENAILLAGE

(30) Priorität: 15.06.2001 DE 10129843
(43) Veröffentlichungstag der Anmeldung: 10.03.2004
(73) Patentinhaber: Humboldt Universität Berlin Charite Universitätsklinikum (CCM), 10117 Berlin (DE); BAM Bundesanstalt für Materialforschung und -prüfung, 12205 Berlin (DE)
(72) Erfinder: MÜLLER, Wolf-Dieter, 12524 Berlin (DE); BERGER, Georg, 16341 Zepernick (DE)
(74) Vertreter: Walter, Wolf-Jürgen
(86) Internationale Anmeldenummer: PCT/DE2002/002229
(87) Internationale Veröffentlichungsnummer: WO 2002/102431

(56) Entgegenhaltungen:
- WO-A-87/07256
- WO-A-91/07357
- DE-A- 4 126 800
- DE-C- 19 744 809

## Beschreibung

Die Erfindung betrifft ein metallisches Implantat, das an seiner Oberfläche ein glasig-kristallines bioaktives Material trägt sowie ein Strahlgut zur Behandlung der Oberfläche.

Anorganische Materialien mit Langzeitstabilität sind an sich bekannt. Auch Werkstoffe, die ihren speziellen Einsatz als bioaktive Knochenersatzwerkstoffe finden und eine ausreichende Langzeitstabilität besitzen, sind in der Literatur beschrieben. Beispielsweise wurde ständig über den erfolgreichen klinischen Einsatz von Glaskeramiken bzw. Sinterglaskeramiken mit den Hauptkristallphasen Apatit und Wollastonit berichtet [Kokubo, T., Biomaterials, 12 (1991)155 - 163; Berger, G. et al.: Long-term stable bioactive glass ceramics an implant material - ten years of clinical experience, Forth World Biomaterial Congress, Berlin, April 24 - 28,1992, Transactions p. 33]. Übertroffen wurde deren chemische Stabilität durch ebenfalls bioaktive Werkstoffe auf der Basis von Calcium-Zirconium/Titan-Phosphat, die sich nur durch keramische Verfahren herstellen lassen, jedoch keine Schmelze bei in der Glasindustrie üblichen Temperaturen (etwa 1650°C) bilden, was bekanntermaßen Nachteile bei der mechanischen Stabilität von derartigen Granulaten und insbesondere daraus hergestellten Formkörpern bewirkt (Biomaterials 18 (1997) 1671 - 1675). Es ist weiterhin bekannt, daß metallische Implantate an ihrer Oberfläche aufgerauht werden, womit die Haftung zum umgebenden Gewebe verbessert werden soll. Bei diesem Abstrahlen werden infolge tribochemischer Reaktionen stets Partikel in die metallische Oberfläche eingebracht.

übliche, kommerzielle Verfahren beziehen sich auf das Strahlen mit Al₂O₃. Die Wirkung hinsichtlich der Aufrauhung der Oberfläche ist sehr gut, hingegen ist die Anreicherung der Oberfläche mit Al₂O₃ unter dem Aspekt der Biokompatibilität zweifelhaft. Eine Verbesserung wurde dadurch erreicht, in dem glaskeramische Werkstoffe eingesetzt werden, die Apatit und Wollastonit bzw. Apatit, Wollastonit und Ca₇Mg₂P₂O₂₄ als Hauptkristallphase beinhalten, wie es in DE 41 26 800 A1 beschrieben wurde. Dabei werden wenigstens 1 µm dicke Teilchen in die Oberfläche des Implantats eingebracht bei einer Härte nach Mohs von 5-7 ° der glaskristallinen oder keramischen Materialien. Die erzeugte Oberflächenrauhigkeit liegt bei 5-10 µm.

Die chemische Langzeitstabilität dieser Werkstoffe im Zusammenhang mit ihrer Bioaktivität (direkter bindegewebsfreier Knochenkontakt) galt bislang als unerreicht.

Langzeitstabile kommerzielle Werkstoffe leiten sich von Apatiten ab. Entweder handelt es sich um Keramiken mit Hydroxyl- oder Fluorapatiten (HAp, FAp) als Hauptkristallphase und schlechten Verarbeitungseigenschaften oder es handelt sich um Glaskeramiken, die meist eine weitere Kristallphase einschließen, z.B. Wollastonit mit dem Ziel verbesserter mechanischer Eigenschaften, Glimmerphasen zur Erzielung einer maschinellen Bearbeitbarkeit etc. Die Verarbeitungseigenschaften von Glaskeramiken gelten im allgemeinen als ausreichend. Die bekannten Kombinationen zwischen Apatiten weiteren Kristallphasen bzw. Restglasanteilen führen unter dem Aspekt der Langzeitstabilität stets zu Werkstoffen mit höherer Löslichkeit als sie bei den reinen Phasen (HAp, FAp) zu bestimmen sind.

Aus der DE 4126800 A1 ist ein Verbundwerkstoff aus Metall bekannt, der zwischen einer Beschichtung aus Kunststoff oder Zement oder glasig-kristallinen Material bestimmte Teilchen auf Basis des Systems CaO-P₂O₅-SiO₂ und gegebenenfalls ZrO₂ mit Apatit und Wollastonit als Hauptkristallphasen enthält.

Der Erfindung liegt die Aufgabe zugrunde, ein metallisches Implantat bereitzustellen, das an seiner Oberfläche aufgerauht ist und ein bioaktives Material trägt und gleich-zeitig eine verbesserte chemische Beständigkeit hat.

Erfindungsgemäß wird ein oberflächenbehandeltes metallisches Implantat bereitgestellt, bei dem ein metallischer Implantat-Grundkörper an seiner Oberfläche Partikel eines bioaktiven glasig-kristallinen Materials trägt, bestehend aus 15 - 45 Gew-% CaO, 40 - 45 Gew-% P₂O₅, 10 - 40 Gew-% ZrO₂ und 0,7 - 3,5 Gew-% Fluorid, und dieses Material als Hauptkristallphasen Apatit und Calciumzirconiumphosphat und als Nebenbestandteil eine Glasphase enthält, wobei die Hauptkristallphasen zusammen wenigstens 35 Gew-% betragen und die Nebenbestandteile 5 bis 15 Gew-% betragen, und wobei alle Prozentangaben auf das Gesamtgewicht des glasig-kristallinen Materials bezogen sind.

Ein bevorzugtes glasig-kristallines Material enthält 23 - 39 Gew-% CaO, 40 - 45 Gew-% P₂O₅, 20 - 35 Gew-% ZrO₂ und 1 - 3 Gew-% Fluorid und enthält als Hauptkristallphasen Apatit und Calciumzirconiumphosphat und als Nebenbestandteil eine Glasphase, wobei die Hauptkristallphasen zusammen wenigstens 35 Gew-% betragen und die Nebenbestandteile 5 bis 15 Gew-% betragen.

Ein ebenfalls bevorzugtes glasig-kristallines Material enthält 23 - 39 Gew-% CaO, 40 - 45 Gew-% P₂O₅, 20 - 35 Gew-% ZrO₂ und 1 - 3 Gew-% Fluorid sowie zusätzlich 0,1 bis 6 Gew-% Na₂O, und es enthält als Hauptkristallphasen Apatit und Calciumzirconiumphosphat und als Nebenbestandteil eine Glasphase und gegebenenfalls zusätzlich als Nebenbestandteil eine Natriumzirconiumphosphat-Phase. Dabei beträgt der Anteil der Hauptkristallphasen zusammen wenigstens 35 Gew-%, und die Nebenbestandteile können jeweils 5 bis 15 Gew-% betragen.

Weiterhin kann das glasig-kristalline Material zusätzlich 0,1 bis 6 Gew-% Magnesiumoxid und/oder Kaliumoxid enthalten und gegebenenfalls zusätzlich die entsprechenden Phasen als Nebenbestandteil.

Der Gehalt an Na₂O, MgO und/oder K₂O liegt vorzugsweise im Bereich von 1 bis 6 Gew-%. Der Anteil der entsprechenden Nebenkristallphase Natriumzirconiumphosphat kann im Bereich von 5 bis 10 Gew-% liegen.

Bei dem Implantat kann ein Teil des (1) bioaktiven glasig-kristallinen Materials ersetzt sein durch (2) ein langzeitstabiles biokompatibles glaskeramisches Material, das Apatit und Wollastonit als Hauptkristallphasen enthält und/oder (3) ein resorbierbares biokompatibles glaskeramisches Material, das Apatit, Wollastonit und Ca₇Mg₂P₂O₂₄ als Hauptkristallphasen enthält. Der Anteil des glasig-kristallinen Materials (1) beträgt 95 bis 40 Gew-%, vorzugsweise 95 bis 60 Gew-%, bezogen auf das Gesamtgewicht des bioaktiven und biokompatiblen Materials. Das Material (1) hat eine Körnung im Bereich von 53 bis 350 µm, das Material (2) eine Körnung im Bereich von 70 bis 315 µm und das Material (3) eine Körnung im Bereich von 53 bis 250 µm.

Die hier verwendeten Begriffe "Glaskeramik" und "glasig-kristallines Material" sind im Allgemeinen nicht immer eindeutig definierbar. Es liegen sowohl kristalline als auch glasige bzw. röntgenamorphe Phasen innig vermischt vor. Für die vorliegende Erfindung ist es ohne Belang, ob eine Phase neben der anderen vorliegt oder ob eine Phase die andere umhüllt.

Als "Hauptkristallphase" wird hier eine kristalline Phase bezeichnet, deren Mengenanteil wenigstens doppelt so groß ist, wie die einer Nebenphase, wobei Konzentrationen um 15% und darunter, vorzugsweise unter 10 Gew-% als Nebenphasen bezeichnet werden.

Es wurde nun gefunden, daß das glasig-kristalline Material, obwohl es Apatit enthält, auch im leicht sauren Medium, wie es bei Entzündungsreaktionen beobachtet wird, nämlich pH = 6,0 (Berger et al., Hydroxyapatite's solubility may cause loosening of coated implants, Bioceramics Vol. 13, edited by Santro Giannini and Antonio Moroni (Proceedings of the 13^{th} International Symposium on Ceramics in Medicine); Trans Tech Publ. Ltd, Swiss, 2000, 111 - 114], sehr lösungsstabil ist. Das heißt, entgegen dem bisherigen Trend der Löslichkeitserhöhung von Apatiten durch zusätzliche Kristallphasen oder Restglasanteile, tritt überraschend bei der erfindungsgemäßen Kombination eine Erniedrigung der Löslichkeit des Materials ein.

Ferner wurde überraschenderweise gefunden, daß nach Lagerung in deionisiertem Wasser der Werkstoff (das glasig-kristalline Material) nach anfänglich alkalischer Reaktion seine Oberflächeneigenschaften in Richtung physiologische pH-Werte (7,4) zu verändert.

Der thermische Ausdehnungskoeffizient des Werkstoffes liegt im Bereich von 27 °C und 300, 400, 600 oder 800 °C zwischen 1,4 und 6•10⁻⁶ grad⁻¹.

Ein weiteres Merkmal des Werkstoffes besteht darin, daß es eine Gesamtlösung von 4 bis 5,5 mg/L hat, wenn die Prüfung in 0,2M TRIS-HCl-Puffer-Lösung bei pH=7,4, T=37 °C, an einer Kornfraktion von 315-400µm, 120h lang bei einem Oberflächen(Probe)-zu-Volumen(Lösungsmittel)-Verhältnis von 5cm⁻¹ erfolgt.

Ein weiteres Merkmal des Werkstoffes besteht darin, daß es bereits nach Wasserlagerung (144h) bei 37 °C die Oberfläche des Werkstoffes so einstellt, daß physiologische pH-Werte um 7,4 bestimmt werden können. Damit ist Stabilität im sauren pH-Bereich zwischen 7,0 und 7,5 gegeben. Erhöht man die Wasserbadtemperatur, so wird die Oberflächenveränderung dementsprechend beschleunigt herbeigeführt.

Wenn das glasig-kristalline Material, das sowohl bioaktiv als auch langzeitstabil ist, nach seiner Herstellung aus der Schmelzphase zu einem Granulat mit einer Körnung von 53 bis 350 im gemahlen und gegebenenfalls abgesiebt wurde, kann mit diesem Granulat als Strahlgut eine Strahlbehandlung der entsprechenden Oberfläche eines Metallimplantats erfolgen.
Infolge tribochemischer Reaktionen werden Teilchen des Granulats in die Oberfläche eingebracht und zugleich wird eine Aufrauhung der Oberfläche erreicht. Da das neue glasig-kristalline Material bioaktiv ist und zugleich eine ausgezeichnete Langzeitstabilität aufweist, wird der Einheilprozeß des Implantats deutlich beschleunigt, und die Gefahr von Komplikationen kann geringer gehalten werden als bei den bekannten Strahlmaterialien.

Die Erfindung betrifft auch ein Strahlgut für metallische Implantate, gekennzeichnet durch ein bioaktives glasig-kristallines Material, bestehend aus
15 - 45 Gew-% CaO, 40 - 45 Gew-% P₂O₅, 10 - 40 Gew-% ZrO₂ und 0, 7 - 3,5 Gew-% Fluorid und enthaltend als Hauptkristallphasen Apatit und Caiciumzirconiumphosphat und als Nebenbestandteil eine Glasphase, wobei die Hauptkristallphasen zusammen wenigstens 35 Gew-% betragen und die Nebenbestandteile 5 bis 15 Gew-% betragen, und wobei alle Prozentangaben auf das Gesamtgewicht des glasig-kristallinen Materials bezogen sind, und mit einer Körnung des glasig-kristallinen Materials von 53 bis 350 µm .

Eine Ausführungsform des Strahlgutes kann darin bestehen, daß das glasig-kristalline Material zusätzlich 0,1 bis 6 Gew-% Na₂O enthält und gegebenenfalls zusätzlich als Nebenbestandteil eine Natriumzirconiumphosphat-Phase.

Eine weitere Ausführungsform des Strahlgutes kann darin bestehen, daß ein Teil des (1) bioaktiven glasig-kristallinen Materials ersetzt ist durch (2) ein langzeitstabiles biokompatibles glaskeramisches Material, das Apatit und Wollastonit als Hauptkristallphasen enthält (z.B. gemäß Patent DD 247574) und/oder (3) ein resorbierbares biokompatibles glaskeramisches Material, das Apatit, Wollastonit und Ca₇Mg₂P₂O₂₄ als Hauptkristallphasen enthält (z.B. gemäß DE 197 44 809 C1, Beispiel 2, oder EP-B-0541546, Zusammensetzung h).

Vorteilhaft hat das Strahlgut gemäß den genannten Ausführungsformen folgende Teilchengrößen:
das Material (1) eine Körnung im Bereich von 53 bis 350 µm,
das Material (2) eine Körnung im Bereich von 70 bis 315 µm und
das Material (3) eine Körnung im Bereich von 53 bis 250 µm, insbesondere 100 bis 150 µm.

Die Bestimmung der Teilchengrößen erfolgte mit der Lasergranulometrie.

Der Implantat-Grundkörper besteht aus einem beliebigen üblichen Implantatmetall, vorzugsweise aus einem Material, das aus der Gruppe ausgewählt ist, bestehend aus Titanium, Titanium-Legierungen, Edelstahl, Co-Cr-Edelstahl und Co-Cr-Mo-Legierungen.

Die Herstellung des glasig-kristallinen Materials erfolgt durch Gemengebildung mit geeignete Substanzen, also mit
15 - 45 Gew-% CaO, 40 - 45 Gew-% P₂O₅, 10 - 40 Gew-% ZrO₂ und 0,7 - 3,5 Gew-% Fluorid. Das Fluorid wird vorzugsweise als CaF₂ eingebracht. Die Gemengebestandteile werden miteinander kombiniert und in geeigneten meist mehrstufigen Temperaturbehandlungsprogrammen (Haltestufen im Bereich von 400 bis 1500 °C) in einem geeigneten Tiegelmaterial, vorzugsweise bestehend aus einer Pt/Rh-Legierung, bei 1550 bis 1650 °C zum Schmelzen gebracht. Die Schmelze wird vergossen, und die erstarrte Schmelze wird je nach Verwendungszweck an der Luft (spontane Abkühlung) oder im Kühlofen auf Raumtemperatur abgekühlt. Danach wird der Werkstoff aufgemahlen.

Die Erfindung wird nachstehend durch Beispiele näher erläutert. Alle Prozentangaben sind auf das Gewicht bezogen, sofern nicht anderes angegeben ist.

### Beispiel 1 Herstellung des glasig-kristallinen Materials Apatit1/CZP1

Es wird ein Gemenge bereitet, das folgender Zusammensetzung entspricht (Code: Apatit/CZP1):
25,88 CaO
28,44 ZrO₂
43,68 P₂O₅
5,00 CaF₂
Dabei erweist es sich als praktikabel, den CaO-Anteil in Form von 62,79 CaHPO₄ einzusetzen und den noch notwendigen P₂O₅-Anteil in Form von 10,51ml einer 85%igen H₃PO₄. Zunächst wird CaHPO₄, ZrO₂ und CaF₂ gut vermischt, danach mit der Phosphorsäure versetzt, nach Reaktion gemörsert, wobei 4h Trockenhaltestufen von 120°C und 170°C eingelegt wurden. Diese Reaktionsgemisch wird in einem Pt/Rh-Tiegel gefüllt und über die 1h Haltestufen 400 und 800°C erhitzt, abgekühlt und anschließend gemörsert. Das so vorbehandelte Material wird nunmehr im Pt/Rh-Tiegel mit jeweils 15min Haltezeit in den Stufen 800, 1000, 1300, 1500 und schließlich 1600°C zum Schmelzen gebracht und sodann bei Raumtemperatur auf eine stahlplatte gegossen.

Ein Teil der Schmelze wurde durch Mahlen in einer Achatmühle zerkleinert, unter 43µm abgesiebt und sodann einer röntgendiffraktographischen Untersuchung unterzogen. Das Ergebnis (Röntgendiffraktogramm) zeigt, daß in dem glasig-kristallinen Produkt die Kristallphasen Apatit (Fluorapatit/Hydroxylapatit) und Calciumzirconiumphosphat [CaZr₄(PO₄)₆] deutlich nachweisbar sind.

Der restliche Teil der Schmelze wird auf eine Körnung von 60-350 µm gebracht.

### Beispiel 2 Herstellung des glasig-kristallinen Materials Apatit1/CZP2

Es wird ein Gemenge nach der Vorschrift des Beispiels 1 hergestellt mit dem Unterschied, daß hier als zusätzliche Komponente Natriumoxid eingebracht wird (Code: Apatit/CZP2). Der Ansatz sieht die Verwendung folgender Gemengebestandteile vor:
59,93 CaHPO₄
27,10 ZrO₂
3,42 Na₂O
5,00 CaF₂ und
9,56ml einer 85%igen H₃PO₄-Säure.
Es wurde wie im Beispiel 1 gearbeitet. Nach der letzten Temperaturhaltestufe wurde die Schmelze aus dem Tiegel auf eine Stahlplatte gegossen.

Ein Teil der Schmelze wurde durch Mahlen in einer Achatmühle zerkleinert, unter 43µm abgesiebt und sodann einer röntgendiffraktographischen Untersuchung unterzogen. Das Ergebnis (Röntgendiffraktogramm) zeigt, das in dem glasig-kristallinen Produkt die Kristallphasen Apatit (Fluorapatit/Hydroxylapatit) und Calciumzirconiumphosphat [CaZr₄(PO₄)₆] und Natriumzirconiumphosphat [NaZr₂(PO₄)₃] nachweisbar sind.

Der restliche Teil der Schmelze wird auf eine Körnung von 60-350 µm gebracht.

### Beispiel 3 Ausdehnungskoeffizienten Apatit/CZP1

Es erfolgte die Herstellung eines glasig-kristallinen Materials nach Beispiel 1 (Apatit/CZP1). Das Material wird durch Mahlen in einer mit Zirconiumoxid ausgekleideten Mühle zerkleinert, so daß sich ein D₅₀-Wert von 8µm ergab. Das gemahlene Gut wird mit einer 5%igen Polyvinylalkohol(PVA)-Lösung im Verhältnis Mahlgut zu PVA-Lösung von 90 : 10 Masse-% versetzt und zu einem Stab mit 4,7kN in einem Preßgesenk verpreßt. Dieser Rohkörper wird gesintert bei einer Temperatur von 1050° C.

An dem auf diese Weise erhaltenen relativ dichten Formkörper wird der thermische Ausdehnungskoeffizient(AK) bestimmt:
AK im Bereich von 27 - 400°C: 1,90*10⁻⁶ grd Celsius⁻¹
AK im Bereich von 50 - 400°C: 1,86*10⁻⁶ grd Celsius⁻¹
AK im Bereich von 30 - 300°C: 1,45*10⁻⁶ grd Celsius⁻¹
AK im Bereich von 30 - 400°C: 1,88*10⁻⁶ grd Celsius⁻¹
AK im Bereich von 30 - 600°C: 2,6*10⁻⁶ grd Celsius⁻¹
AK im Bereich von 30 - 800°C: 3,2*10⁻⁶ grd Celsius⁻¹

### Beispiel 4 Chemische Beständigkeit Apatit/CZP1 im basischen Bereich

Es erfolgt die Herstellung eines glasig-kristallinen Materials nach Beispiel 1 (Apatit/CZP1). Sodann wird das Material gemörsert und eine Kornfraktion von 315 - 400*µ*m daraus hergestellt.

Das auf diese Weise erhaltene Granulat wird im Vergleich zu einem Grundglas (Ap40_{Glas}) und einer aus diesem Grundglas hergestellten Glaskeramik auf der Basis von Apatit und Wollastonit (Ap40_{krist.}) [chemische Zusammensetzung also identisch zu (Gewichts-%): 44,3 SiO₂; 11,3 P₂O₅; 31,9 CaO; 4,6 Na₂O; 0,19 K₂O; 2,82 MgO und 4,99 CaF₂] hinsichtlich seiner chemischen Beständigkeit verglichen.

Zu diesem Zweck wurden zunächst die spezifischen Oberflächen nach BET mit Krypton als Meßgas bestimmt zu:
Apatit/CZP1: 0,364m²/g
Ap40_{Glas}:0,018 m²/g
Ap40_{krist.}: 0,055m²/g.

Hier zeigt sich, daß das erfindungsgemäße Material eine gewisse offene Porosität im Vergleich zum Grundglas und der daraus hergestellten Glaskeramik aufweist. Diesen Unterschieden wird bei den Lösungsuntersuchungen dadurch Rechnung getragen, indem das Oberflächen(Proben) zu Lösungsmittelvolumen(TRIS-HCl-Puffer-Lösung) konstant auf 5cm⁻¹ eingestellt wird.

Als Lösungsmittel wurde eine 0,2M TRIS-HCl-Puffer-Lösung mit pH=7,4 bei 37°C verwendet. Die Lagerung erfolgte bei 37°C über eine Zeitdauer von 120h. Danach wurde die Gesamtlöslichkeit durch Bestimmung der Einzelionen (Ca, P, Zr) in der Lösung mit Hilfe einer ICP-Messung bestimmt zu:
Apatit/CZP1: 4,1 - 5,1 mg/L
Ap40_{Glas}: 318 - 320mg/L
Ap40_{krist.}: 75,2 - 82,0 mg/L.

Die Werte belegen eindrucksvoll die hohe chemische Beständigkeit des erfindungsgemäßen Werkstoffes unter simulierten physiologischen Bedingungen, einer bekannten Methode zur Bestimmung der Langzeitbeständigkeit in vitro.

### Beispiel 5 Chemische Beständigkeit Apatit/CZP1 im sauren Bereich

Es wird vorgegangen wie im Beispiel 4, jedoch wird eine 0,2M TRIS-HCl-Puffer-Lösung mit dem pH-Wert von 6,0 bei 37°C zur Messung verwendet. Auf diese Weise läßt sich der Fall simulieren, daß infolge einer Wundheilungs- oder Spätinfektion der pH-Wert von den physiologischen 7,4 in den sauren Bereich abgleitet.

Mit Hilfe der ICP wurden nun folgende Werte der Gesamtlösung (Ca, P, Zr) bestimmt:
Apatit/CZP1: 16-19 mg/L
Ap40_{Glas}: 505-518 mg/L
Ap40ₖᵣᵢₛₜ: 117-125 mg/L.

Die Werte belegen eindrucksvoll die hohe chemische Beständigkeit des für die Erfindung eingesetzten Werkstoffes unter simulierten Bedingungen, wie sie bei einer Entzündungsreaktion vorliegen. Demzufolge ist die Erhöhung der absoluten Werte der Löslichkeit für den erfindungsgemäßen Werkstoff wesentlich geringer im Vergleich zu der doch dramatischen Erhöhung im Falle des Grundglases bzw. der Glaskeramik auf Apatit/Wollastonit-Basis.

### Beispiel 6 bis 11

Es wurden Metallimplantate auf Basis von Titanium (Ti) und einer Cobalt-Basislegierung (Wisil) mit Strahlgut behandelt unter den folgenden Bedingungen:
Strahlzeit je cm² Oberfläche: 10-15 sec
Strahldruck: 4 bar
Strahlabstand : 1 cm
Strahlgut :
   a) Material nach Beispiel 1 Korngröße 100-250 µm
   b) Material nach Beispiel 1 Korngröße 100-250 µm + Ap40 Korngröße 100-250 µm (Anteil 30 Gew-%)
   c) Material nach Beispiel 1 Korngröße 100-250 µm + resorbierbare Keramik Korngröße 53-150 µm.
Die Rauhheitszunahme (in µm) der jeweiligen Oberfläche wurde durch Abtasten mit einem Hommeltester ermittelt. Die Ergebnisse sind als Mittelwerte in der folgenden Tabelle aufgeführt.

| Beispiel | 6 Ti | 7 Wisil | 8 Ti | 9 Wisil | 10 Ti | 11 Wisil |
|---|---|---|---|---|---|---|
| Material a | 8,9 | 7,6 | - | - | - | - |
| Material b | - | - | 9,5 | 7,8 | - | - |
| Material c | - | - | - | - | 8,7 | 7,6 |

REM und Elektronenstrahl-Mikroanalyse:
Bei der Aufnahme von EDS-Spektren wurde eine Zunahme des Gehaltes an Bestandteilen aus glasig-kristallinem oder keramischem Material (Ca, P, ...) festgestellt. Die Einlagerung von Partikeln in der Metalloberfläche wurde nachgewiesen.

Es wurde eine elektrochemische Analyse der Metalloberflächen durch Aufnahme von I-E-Kurven (Strom-Spannung) durchgeführt. Im Falle von Ti wurde eine kathodische Verschiebung des Nullstrompotentials im vergleich zu einer unbehandelten Ti-Oberfläche beobachtet.

## Patentansprüche

1. Oberflächenbehandeltes metallisches Implantat, **dadurch gekennzeichnet, daß** ein metallischer Implantat-Grundkörper an seiner Oberfläche Partikel eines bioaktiven glasig-kristallinen Materials trägt, bestehend aus 15 - 45 Gew-% CaO, 40 - 45 Gew-% P₂O₅, 10 - 40 Gew-% ZrO₂ und 0,7 - 3,5 Gew-% Fluorid und als Hauptkristallphasen Apatit und Calciumzirconiumphosphat und als Nebenbestandteil eine Glasphase aufweisend, worin die Hauptkristallphasen des glasig-kristallinen Materials zusammen wenigstens 35 Gew-% betragen und die Nebenbestandteile 5 bis 15 Gew-% betragen, und wobei alle Prozentangaben auf das Gesamtgewicht des glasig-kristallinen Materials bezogen sind, und wobei die Korngröße des glasig-kristallinen Materials im Bereich von 53 bis 350 µm liegt.

2. Implantat nach Anspruch 1, **dadurch gekennzeichnet, daß** es an seiner Oberfläche Partikel eines glasig-kristallinen Materials trägt, bestehend aus 23 - 39 Gew-% CaO, 40 - 45 Gew-% P₂O₅, 20 - 35 Gew-% ZrO₂ und 1 - 3 Gew-% Fluorid.

3. Implantat nach Anspruch 1, **dadurch gekennzeichnet, daß** es
an seiner Oberfläche Partikel eines glasig-kristallinen Materials trägt, die zusätzlich 0,1 bis 6 Gew-% Na₂O enthalten und zusätzlich als Nebenbestandteil eine Natriumzirconiumphosphat-Phase enthalten.

4. Implantat nach Anspruch 1, **dadurch gekennzeichnet, daß** es
an seiner Oberfläche Partikel eines glasig-kristallinen Materials trägt, die zusätzlich 0,1 bis 6 Gew-% Magnesiumoxid und/oder Kaliumoxid enthalten und gegebenenfalls zusätzlich die entsprechenden Phasen als Nebenbestandteil.

5. Implantat nach Anspruch 1, **dadurch gekennzeichnet, daß** der Implantat-Grundkörper aus einem Material besteht, das aus der Gruppe ausgewählt ist, bestehend aus Titanium, Titanium-Legierungen, Edelstahl, Co-Cr-Edelstahl und Co-Cr-Mo-Legierungen.

6. Strahlgut für metallische Implantate, **gekennzeichnet durch** ein bioaktives glasig-kristallines Material, bestehend aus
15 - 45 Gew-% CaO, 40 - 45 Gew-% P₂O₅, 10 - 40 Gew-% ZrO₂ und 0,7 - 3,5 Gew-% Fluorid und enthaltend als Hauptkristallphasen Apatit und Calciumzirconiumphosphat und als Nebenbestandteil eine Glasphase, wobei die Hauptkristallphasen zusammen wenigstens 35 Gew-% betragen und die Nebenbestandteile 5 bis 15 Gew-% betragen, und wobei alle Prozentangaben auf das Gesamtgewicht des glasig-kristallinen Materials bezogen sind, und mit einer Korngröße des glasig-kristallinen Materials von 53 bis 350 µm.

7. Strahlgut nach Anspruch 6, **dadurch gekennzeichnet, daß** das glasig-kristalline Material zusätzlich 0,1 bis 6 Gew-% Na₂O enthält und zusätzlich als Nebenbestandteil eine Natriumzirconiumphosphat-Phase.

## Claims

1. A surface-treated metallic implant comprising a metallic implant base body which has on its surface particles of a bioactive vitreous-crystalline material which comprises 15-45 wt.-% CaO, 40-45 wt.-% P₂O₅, 10-40 wt.-% ZrO₂ and 0.7-3.5 wt.-% fluoride, comprising apatite and calcium zirconium phosphate as main crystal phases and a glass phase as an auxiliary component, wherein the main crystal phases of the vitreous-crystalline material jointly make up at least 35 wt.-% and the auxiliary components make up 5-15 wt.-% and wherein all of the percentage data are in relation to the total weight of the vitreous-crystalline material and the particle size of the vitreous-crystalline material ranges between 53 to 350 µm.

2. An implant according to Claim 1 wherein said implant has on its surface particles of a vitreous-crystalline material consisting of 23-39 wt.-% CaO, 40-45 wt.-% P₂O₅, 20-35 wt.-% ZrO₂ and 1-3 wt.-% fluoride.

3. An implant according to Claim 1 wherein said implant has on its surface particles of vitreous-crystalline material additionally containing 0.1 to 6 wt.-% Na₂O and additionally containing a sodium zirconium phosphate phase as an auxiliary component.

4. An implant according to Claim 1 wherein said implant has on its surface particles of a vitreous-crystalline material additionally containing 0.1 to 6 wt.-% magnesium oxide and/or potassium oxide and, if appropriate, additionally containing the respective phases as an auxiliary component.

5. An implant according to Claim 1 wherein the implant base body consists of a material selected from the group consisting of titanium, titanium alloys, special steel, Co-Cr special steel and Co-Cr-Mo alloys.

6. A blasting material for metallic implants comprising a bioactive vitreous-crystalline material consisting of 15-45 wt.-% CaO, 40-45 wt.-% P₂O₅, 10-40 wt.-% ZrO₂ and 0.7-3.5 wt.-% fluoride, containing apatite and calcium zirconium phosphate as main crystal phases and a glass phase as an auxiliary component, wherein the main crystal phases jointly make up at least 35 wt.-% and the auxiliary components make up 5-15 wt.-% and wherein all of the percentage data are expressed in relation to the total weight of the vitreous-crystalline material and the particle size of the vitreous-crystalline material ranges between 53 and 350 µm.

7. A blasting material according to Claim 6 wherein the vitreous-crystalline material additionally contains 0.1 to 6 wt.-% Na₂O and in addition contains a sodium zirconium phosphate phase as an auxiliary component.

## Revendications

1. Implant métallique qui a reçu un traitement de surface, **caractérisé en ce qu'**un corps de base de l'implant métallique supporte sur sa surface des particules d'un matériau bioactif vitrocristallin, constitué de 15 à 45 % en masse de CaO, 40 à 45 % en masse de P₂O₅, 10 à 40 % en masse de ZrO₂ et 0,7 à 3,5 % en masse de fluorure et comme phases cristallines principales de l'apatite et du phosphate de zirconium et calcium et comme composant minoritaire une phase vitreuse, dans laquelle les phases cristallines principales du matériau vitrocristallin sont conjointement au moins de 35 % en masse et les composants minoritaires sont au moins de 5 à 15 % en masse, et toutes les données en pourcentage étant liées au poids total du matériau vitrocristallin, et la grosseur de grain du matériau vitrocristallin se situant dans la plage de 53 à 350 µm.

2. Implant selon la revendication 1, **caractérisé en ce qu'**il porte sur sa surface des particules d'un matériau vitrocristallin, constitué de 23 à 39 % en masse de CaO, 40 à 45 % en masse de P₂O₅, 20 à 35 % en masse de ZrO_{z} et 1 à 3 % en masse de fluorure.

3. Implant selon la revendication 1, **caractérisé en ce qu'**il porte sur sa surface des particules d'un matériau vitrocristallin qui contiennent en plus 0,1 à 6 % en masse de Na₂O et contiennent en plus comme composant minoritaire une phase de phosphate de zirconium et sodium.

4. Implant selon la revendication 1, **caractérisé en ce qu'**il porte sur sa surface des particules d'un matériau vitrocristallin qui contiennent en plus 0,1 à 6 % en masse d'oxyde de magnésium et/ou d'oxyde de potassium et éventuellement en plus les phases correspondantes comme composant minoritaire.

5. Implant selon la revendication 1, **caractérisé en ce que** le corps de base de l'implant est constitué d'un matériau qui est sélectionné parmi le groupe constitué du titane, des alliages de titane, d'acier spécial, d'acier spécial de Co-Cr et d'alliages de Co-Cr-Mo.

6. Abrasif pour implants métalliques, **caractérisé par** un matériau vitrocristallin bioactif, constitué de
15 à 45 % en masse de CaO, 40 à 45 % en masse de P₂O₅, 10 à 40 % en masse de ZrO₂ et 0,7 à 3,5 % en masse de fluorure et contenant comme phases cristallines principales de l'apatite et du phosphate de zirconium et calcium et comme composant minoritaire une phase vitreuse, les phases cristallines principales étant conjointement d'au moins 35 % en masse et les composants minoritaires étant de 5 à 15 % en masse, et toutes les données en pourcentage étant liées au poids total du matériau vitrocristallin, et avec une grosseur de grain du matériau vitrocristallin de 53 à 350 µm.

7. Abrasif selon la revendication 6, **caractérisé en ce que** le matériau vitrocristallin contient en plus 0,1 à 6 % en masse de Na₂O et en outre une phase de phosphate et zirconium comme composant minoritaire.
